# EUROPEAN PATENT SPECIFICATION

(11) **EP 0 916 640 B1**
(45) Date of publication and mention of the grant of the patent: **12.06.2002**
(21) Application number: 98309162.0
(22) Date of filing: 09.11.1998
(51) Int. Cl.: C07C 43/29, A01N 31/14, C07C 43/295, C07C 43/225, C07C 25/24

(54) **1,4-diaryl-2,3-difluoro-2-butene insecticidal and acaricidal agents**
Insektizide und akarizide 1,4-Diaryl-2,3-difluor-2-butene
1,4-Diaryl-2,3-difluoro-2-butènes comme agents insecticides et acaricides

(30) Priority: 12.11.1997 US 969056
(43) Date of publication of application: 19.05.1999
(73) Proprietor: BASF AKTIENGESELLSCHAFT, 67056 Ludwigshafen (DE)
(72) Inventor: Bentley, Terence James, East Windsor, New Jersey 08561 (US); Barnes, Keith Douglas, Newtown, Pennsylvania 18940 (US)
(74) Representative: Langfinger, Klaus Dieter, Dr.

(56) References cited:
- EP-A- 0 811 593
- WO-A-94/06741
- WO-A-97/16067
- GB-A- 2 288 803
- US-A- 5 248 834
- D. J. BURTON: "Difluoromethylene chain-extension reactions. Preparation of fluorinated alkenes and alkadienes from olefin precursors" JOURNAL OF THE AMERICAN CHEMICAL SOCIETY, vol. 102, no. 11, 21 May 1980, pages 3980-3982, XP002093857 DC US

## Description

Insect and acarid pests destroy growing and harvested crops. In the United States, agronomic crops must compete with thousands of those pests. In particular tobacco budworms, southern armyworms and corn rootworms are especially devastating to crops.

In spite of the commercial insecticides and acaricides available today, damage to crops, both growing and harvested, caused by insect and acarid pests still occurs. Accordingly, there is ongoing research to create new and more effective insecticidal and acaricidal agents.

Certain fluoroolefin compounds are known to possess insecticidal and acaricidal activity (see, e.g. U.S. 5,248,834; GB-A 2,288,803; WO-A 94/06741 and WO-A 97/16067 and later published U.S. 5,998,673 and EP-A 811 593).

A phenylfluorobutene compound useful as intermediate in the preparation of diphenylfluoroolefins has been disclosed in JACS, vol. 102, 3980 (1980).

However, the fluoroolefin compounds disclosed in those patents and patent applications are outside the scope of the present invention. U.S. 5,248,834 generically discloses certain 1-aryl-1-(3-aryl-1,2-difluoroprop-1-enyl)cyclopropane compounds. However, that patent does not provide a method to prepare those compounds. In fact, U.S. 5,248,834 does not provide a method to prepare any fluoroolefin compounds.

It is, therefore, an object of the present invention to provide compounds which are highly effective for the control of insect and acarid pests.

It is also an object of the present invention to provide a method for the control of insect and acarid pests.

It is a further object of this invention to provide a method for the protection of growing and harvested crops from damage caused by insect and acarid attack and infestation.

Those and other objects of the present invention will become more apparent from the detailed description thereof set forth below.

The present invention comprises 1,4-diaryl-2,3-difluoro-2-butene compounds which are useful as insecticidal and acaricidal agents. Those compounds are also useful for protecting plants from damage caused by insect and acarid attack and infestation.

The 1,4-diaryl-2,3-difluoro-2-butene compounds of the present invention have the structural formula I 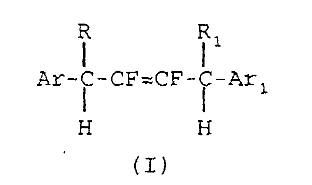 wherein
- Ar: is phenyl optionally substituted with any combination of from one to three halogen, C₁-C₄alkyl, C₁-C₄haloalkyl, C₁-C₄alkoxy, C₁-C₄haloalkoxy or hydroxy groups,
1- or 2-naphthyl optionally substituted with any combination of from one to three halogen, C₁-C₄alkyl, C₁-C₄haloalkyl, C₁-C₄alkoxy or C₁-C₄haloalkoxy groups, or
a 5- or 6-membered heteroaromatic ring optionally substituted with any combination of from one to three halogen, C₁-C₄alkyl, C₁-C₄haloalkyl, C₁-C₄alkoxy or C₁-C₄haloalkoxy groups;
- R: is hydrogen, C₁-C₄alkyl, C₁-C₄haloalkyl, C₃-C₆cycloalkyl or C₃-C₆halocycloalkyl;
- R₁: is hydrogen, F, Cl, Br, cyano or OR₂,
- R₂: is hydrogen or C₁-C₄alkyl; and
- Ar₁: is phenoxyphenyl optionally substituted with any combination of from one to six halogen, C₁-C₄alkyl, C₁-C₄haloalkyl, C₁-C₄alkoxy or C₁-C₄haloalkoxy groups,
phenyl optionally substituted with any combination of from one to five halogen, C₁-C₄alkyl, C₁-C₄haloalkyl, C₁-C₄alkoxy or C₁-C₄haloalkoxy groups,
biphenyl optionally substituted with any combination of from one to five halogen, C₁-C₄alkyl, C₁-C₄haloalkyl, C₁-C₄alkoxy or C₁-C₄haloalkoxy groups,
phenoxypyridyl optionally substituted with any combination of from one to five halogen, C₁-C₄alkyl, C₁-C₄haloalkyl; C₁-C₄alkoxy or C₁-C₄haloalkoxy groups,
benzylpyridyl optionally substituted with any combination of from one to five halogen, C₁-C₄alkyl, C₁-C₄haloalkyl, C₁-C₄alkoxy or C₁-C₄haloalkoxy groups,
benzylphenyl optionally substituted with any combination of from one to five halogen, C₁-C₄alkyl, C₁-C₄haloalkyl, C₁-C₄alkoxy or C₁-C₄haloalkoxy groups,
benzoylphenyl optionally substituted with any combination of from one to five halogen, C₁-C₄alkyl, C₁-C₄haloalkyl, C₁-C₄alkoxy or C₁-C₄haloalkoxy groups,
1- or 2-naphthyl optionally substituted with any combination of from one to three halogen, C₁-C₄alkyl, C₁-C₄haloalkyl, C₁-C₄alkoxy or C₁-C₄haloalkoxy groups, or
a 5- or 6-membered heteroaromatic ring optionally substituted with any combination of from one to three halogen, C₁-C₄alkyl, C₁-C₄haloalkyl, C₁-C₄alkoxy or C₁-C₄haloalkoxy groups, and
the optical isomers thereof, and
the (E)- and (Z)- isomers thereof.

This invention also comprises compositions containing those compounds and methods for using those compounds and compositions. Advantageously, it has been found that the 1,4-diaryl-2,3-difluoro-2-butene compounds of the present invention, and compositions containing them, are useful for the control of insect and acarid pests. The compounds of this invention are also useful for the protection of plants from damage caused by insect and acarid attack and infestation.

The present invention provides a method for the control of insect or acarid pests which comprises contacting said pests or their food supply, habitat or breeding grounds with a pesticidally effective amount of a 1,4-diaryl-2,3-difluoro-2-butene compound of formula I.

The present invention also provides a method for the protection of growing plants from attack or infestation by insect or acarid pests which comprises applying to the foliage of the plants, or to the soil or water in which they are growing, a pesticidally effective amount of a 1,4-diaryl-2,3-difluoro-2-butene compound of formula I.

The 1,4-diaryl-2,3-difluoro-2-butene compounds of the present invention have the structural formula I 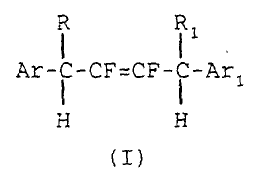 wherein Ar, Ar₁, R and R₁ are as described hereinabove for formula I.

In formula I above, 5- and 6-membered heteroaromatic rings include, but are not limited to, pyridyl, pyrazolyl, imidazolyl, triazolyl, isoxazolyl, tetrazolyl, pyrazinyl, pyridazinyl, triazinyl, furanyl, thienyl and thiazolyl rings each optionally substituted as described in formula I above.

Exemplary of halogen hereinabove are fluorine, chlorine, bromine and iodine. The terms "C₁-C₄haloalkyl" "C₃-C₆halocycloalkyl" and "C₁-C₄haloalkoxy" are defined as a C₁-C₄alkyl group, a C₃-C₆cycloalkyl group and a C₁-C₄alkoxy group substituted with one or more halogen atoms, respectively.

When used herein as a group or part of a group the term alkyl includes straight or branched chain alkyl groups such as methyl, ethyl, n-propyl, isopropyl, n-butyl, isobutyl, s-butyl and t-butyl. When used herein as a group or part of a group the term cycloalkyl includes cyclopropyl, cyclobutyl, cyclopentyl and cyclohexyl.

Groups containing two or more rings, such as phenoxyphenyl, biphenyl, phenoxypyridyl and benzylphenyl, which may be substituted may be substituted on either ring unless otherwise specified herein.

Ar is preferably phenyl optionally substituted with any combination of from one to three halogen, C₁-C₄alkyl, C₁-C₄haloalkyl, C₁-C₄alkoxy or C₁-C₄haloalkoxy groups, more preferably substituted with any combination of from one to three of hydroxy, methoxy, ethoxy, difluoromethoxy, trifluoromethoxy, chlorine and fluorine, most preferably a mono substituted phenyl such as para substituted phenyl.

R is preferably C₁-C₄alkyl, C₁-C₄haloalkyl or C₃-C₆cycloalkyl or C₃-C₆halocycloalkyl, more preferably isopropyl, trifluoromethyl or cyclopropyl, most preferably isopropyl. R₁ is preferably hydrogen.

Ar₁ is preferably 3-phenoxyphenyl, 3-biphenyl or 3-benzylphenyl each optionally substituted with any combination of from one to five halogen, C₁-C₄alkyl, C₁-C₄haloalkyl, C₁-C₄alkoxy or C₁-C₄haloalkoxy groups, more preferably 3-phenoxyphenyl optionally substituted with any combination of from one to six halogen, C₁-C₄alkyl, C₁-C₄haloalkyl, C₁-C₄alkoxy or C₁-C₄haloalkoxy groups. The most preferred substituent being a halogen, particularly fluorine. Particular values of Ar₁ are 3-phenoxyphenyl and 4-fluoro-3-phenoxyphenyl.

Preferred formula I pesticidal agents of this invention are those wherein
- Ar: is phenyl optionally substituted with any combination of from one to three halogen, C₁-C₄alkyl, C₁-C₄haloalkyl, C₁-C₄alkoxy or C₁-C₄haloalkoxy groups;
- R: is C₁-C₄alkyl, C₁-C₄haloalkyl, C₃-C₆cycloalkyl or C₃-C₆halocycloalkyl;
- R₁: is hydrogen, F, Cl, Br, cyano or OR₂,
- R₂: is hydrogen or C₁-C₄alkyl; and
- Ar₁: is 3-phenoxyphenyl optionally substituted with any combination of from one to six halogen, C₁-C₄alkyl, C₁-C₄haloalkyl, C₁-C₄alkoxy or C₁-C₄haloalkoxy groups,
3-biphenyl optionally substituted with any combination of from one to five halogen, C₁-C₄alkyl, C₁-C₄haloalkyl, C₁-C₄alkoxy or C₁-C₄haloalkoxy groups, or
3-benzylphenyl optionally substituted with any combination of from one to five halogen, C₁-C₄alkyl, C₁-C₄haloalkyl, C₁-C₄alkoxy or C₁-C₄haloalkoxy groups.

More preferred 1,4-diaryl-2,3-difluoro-2-butene compounds of this invention are those wherein
- Ar: is phenyl optionally substituted with any combination of from one to three halogen, C₁-C₄alkyl, C₁-C₄haloalkyl, C₁-C₄alkoxy or C₁-C₄haloalkoxy groups;
- R: is isopropyl, trifluoromethyl or cyclopropyl;
- R₁: is hydrogen; and
- Ar₁: is 3-phenoxyphenyl optionally substituted with any combination of from one to six halogen, C₁-C₄alkyl, C₁-C₄haloalkyl, C₁-C₄alkoxy or C₁-C₄haloalkoxy groups.

Most preferred insecticidal and acaricidal agents of the present invention are those wherein
- Ar: is phenyl optionally substituted with any combination of from one to three halogen, C₁-C₄alkyl, C₁-C₄haloalkyl, C₁-C₄alkoxy or C₁-C₄haloalkoxy groups;
- R: is isopropyl;
- R₁: is hydrogen; and
- Ar₁: is 3-phenoxyphenyl optionally substituted with any combination of from one to six halogen, C₁-C₄alkyl, C₁-C₄haloalkyl, C₁-C₄alkoxy or C₁-C₄haloalkoxy groups.

Formula I compounds of this invention which are particularly effective insecticidal agents include
4-(*p*-chlorophenyl)-2,3-difluoro-5-methyl-1-(*m*-phenoxyphenyl)-2-hexene, (E)-;
4-(*p*-chlorophenyl)-2,3-difluoro-1-(4-fluoro-3-phenoxyphenyl)-5-methyl-2-hexene, (E)-;
2,3-difluoro-5-methyl-1-(*m*-phenoxyphenyl)-4-[*p*-(trifluoromethoxy)phenyl]-2-hexene, (E)-;
4-(*p*-ethoxyphenyl)-2,3-difluoro-1-(*m*-phenoxyphenyl)-5-methyl-2-hexene, (E)-;
4-(*p*-ethoxyphenyl)-2,3-difluoro-1-(4-fluoro-3-phenoxyphenyl)-5-methyl-2-hexene, (E)-;
2,3-difluoro-1-(4-fluoro-3-phenoxyphenyl)-5-methyl-4-[*p*-(trifluoromethoxy)phenyl]-2-hexene, (E)-;
2,3-difluoro-4-(*p*-fluorophenyl)-5-methyl-1-(*m*-phenoxyphenyl)-2-hexene, (E)-; and
2,3-difluoro-1-(4-fluoro-3-phenoxyphenyl)-4-(*p*-fluorophenyl)-5-methyl-2-hexene, (E)-, among others.

The 1,4-diaryl-2,3-difluoro-2-butene compounds of this invention are especially useful for the control of tobacco budworms, southern armyworms and corn rootworms.

In a preferred embodiment of the present invention, the fluorine atoms attached to the carbon atoms of the double bond in the formula I compounds are in the (E)-configuration with respect to each other.

The present invention also provides a process for the preparation of a compound of formula I as described above which comprises:
a) converting a compound of formula III to a desired compound of formula I wherein R₁ is H, or OR₂ and R₂ is hydrogen, 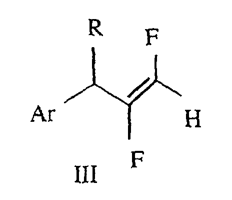 for example by reacting a compound of formula III with an alkyllithium such as n-butyllithium, zinc chloride, tetrakis(triphenyl phosphine)palladium(0),
   or by reacting a compound of formula III with an alkyllithium such as n-butyllithium and an aldehyde of formula Ar₁CHO wherein Ar₁ is as defined above
   to provide a compound of formula I wherein R₁ is OR₂ and R₂ is hydrogen;
b) converting a compound of formula I wherein R₁ is H, or OR₂ and R₂ is hydrogen to a desired compound of formula I wherein R₁ is F, Cl or Br,
   for example by reacting a formula I compound wherein R₁ is OR₂ and R₂ is hydrogen with thionyl chloride, thionyl bromide or diethylaminosulfur trifluoride
   or by halogenating a compound of formula I wherein R₁ is hydrogen with a chlorinating agent such as N-chloro-succinimide or a brominating agent such as N-bromo-succinimide;
c) converting a compound of formula I wherein R₁ is F, Cl or Br to a desired compound of formula I wherein R₁ is CN or OR₂ and R₂ is hydrogen or C₁-C₄alkyl,
   for example by reacting the compound of formula I wherein R₁ is F, Cl or Br with sodium bromide to provide a compound of formula I wherein R₁ is CN,
   or by reacting the compound of formula I wherein R₁ is F, Cl or Br with a compound of formula NaOR₄ wherein R4 is C₁-C₄alkyl to provide a compound of formula I wherein R₁ is OR₂ and R₂ is C₁-C₄alkyl,
   or by reacting the compound of formula I wherein R₁ is F, Cl or Br with sodium hydroxide in water to provide a compound of formula I wherein R₁ is OR₂ and R₂ is hydrogen;
or d) converting a compound of formula VI wherein R₃ is SO₂CH₃ or SO₂C₆H₄-4-CH₃ to a desired compound of formula I wherein R₂ is CN 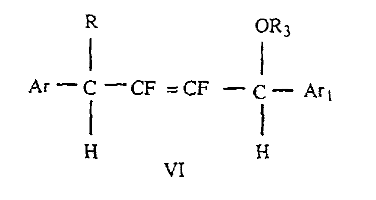 for example by reacting a compound of formula VI with sodium cyanide.

Formula I compounds wherein R₁ is hydrogen may be prepared, as illustrated in Flow Diagram I, by reacting a 3-aryl-1,1,2-trifluoro-1-propene compound of formula II with sodium bis(2-methoxyethoxy)aluminum hydride and a mineral acid such as hydrochloric acid to form a 3-aryl-1,2-difluoro-1-propene compound of formula III, and sequentially reacting the formula III compound with an alkyllithium such as *n*-butyllithium, zinc chloride, tetrakis(triphenylphosphine)palladium(0) and a substituted methyl halide of formula IV.

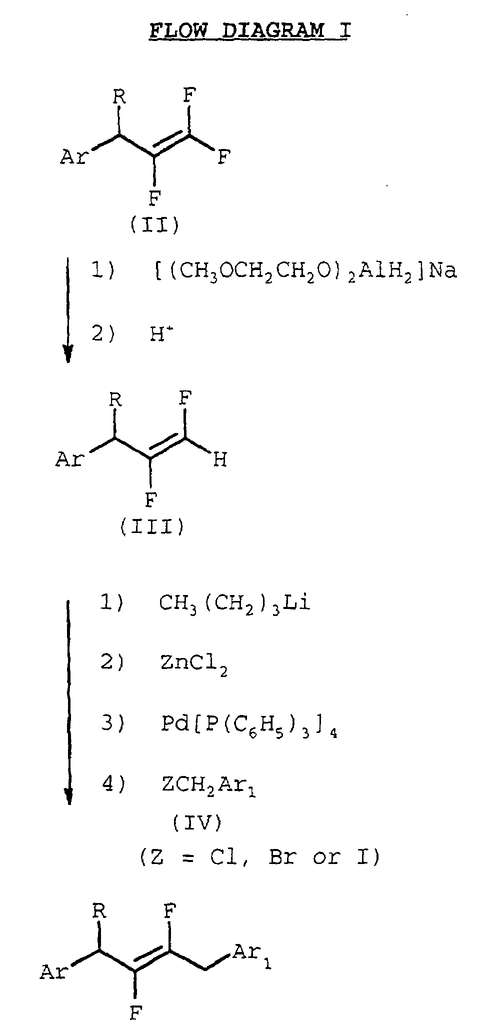

Formula I compounds wherein R₁ is hydroxy may be prepared, as shown in Flow Diagram II, by reacting a 3-aryl-1,2-difluoro-1-propene compound of formula III with an alkyllithium such as *n*-butyllithium and an aldehyde of formula V.

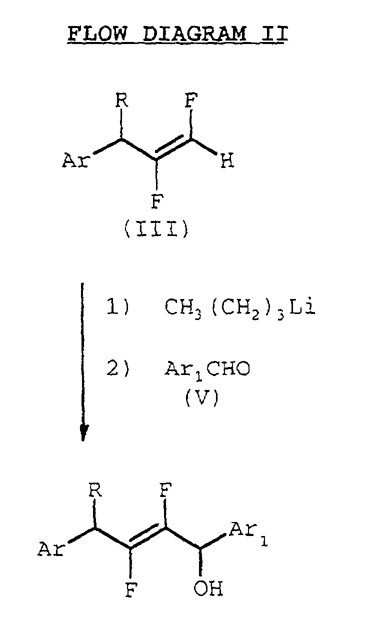

Formula I compounds wherein the fluorine atoms about the double bond are in the (Z)- configuration may be prepared by isomerizing the formula III compounds described hereinabove which are predominately in the (E)-configuration using conventional procedures such as exposure to light.

Compounds of formula I wherein R₁ is F, Cl or Br may be prepared by reacting a formula I compound wherein R₁is hydroxy with thionyl chloride, thionyl bromide or diethylaminosulfur trifluoride. The reaction scheme is shown below in Flow Diagram III.

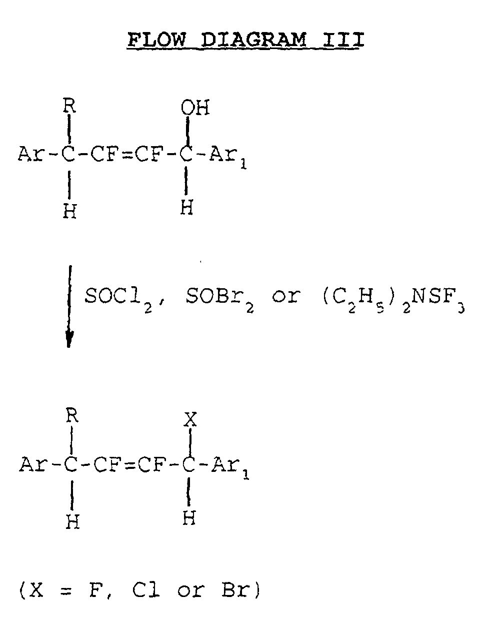

Alternatively, formula I compounds wherein R₁is Cl or Br may be prepared by halogenating a formula I compound wherein R₁ is hydrogen with a chlorinating agent such as N-chlorosuccinimide or a brominating agent such as N-bromosuccinimide. The reaction scheme is shown below in Flow Diagram IV.

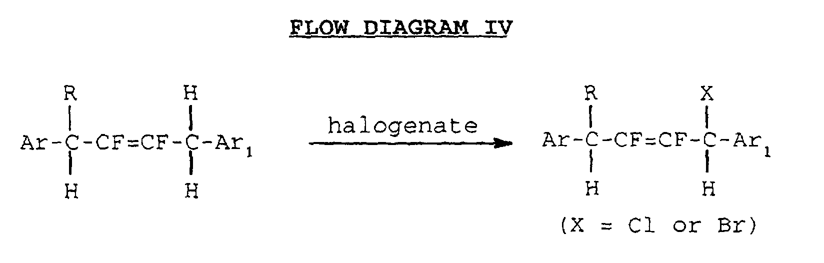

Formula I compounds wherein R₁ is cyano may be prepared by reacting a formula I compound wherein R₁ is F, Cl or Br with sodium cyanide. The reaction scheme is shown in Flow Diagram V.

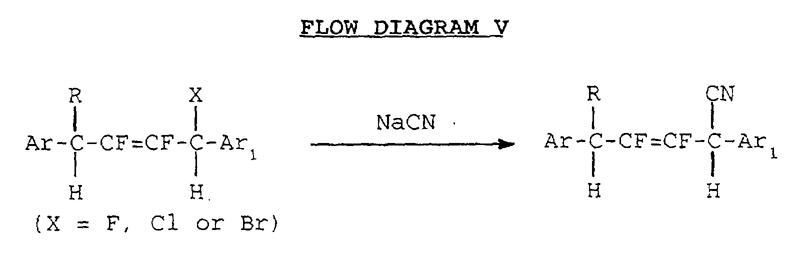

Alternatively, formula I compounds wherein R₁is cyano may be prepared, as illustrated in Flow Diagram VI, by reacting a formula I compound wherein R₁ is hydroxy with methanesulfonyl chloride (mesyl chloride) or *p*-toluene-sulfonyl chloride (tosyl chloride) in the presence of a base to form an intermediate compound of formula VI, and reacting the intermediate compound with sodium cyanide.

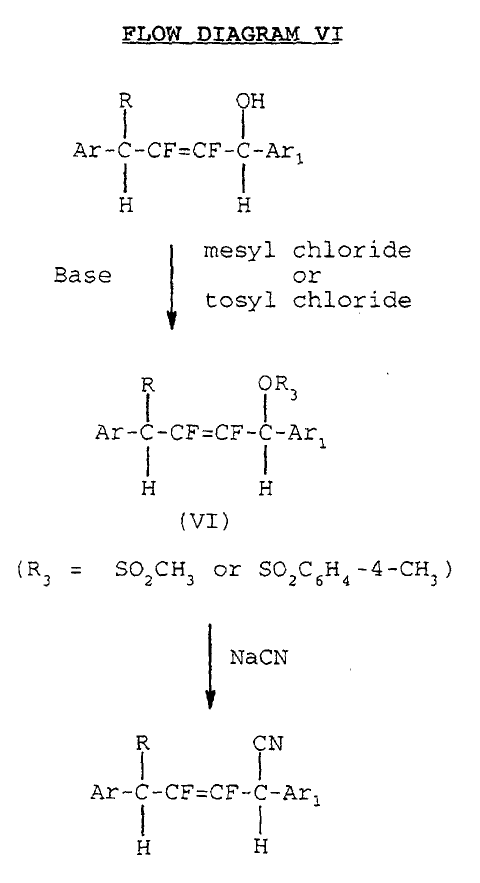

Formula I compounds wherein R₁ is OR₂ may be prepared as shown below in Flow Diagram VII.

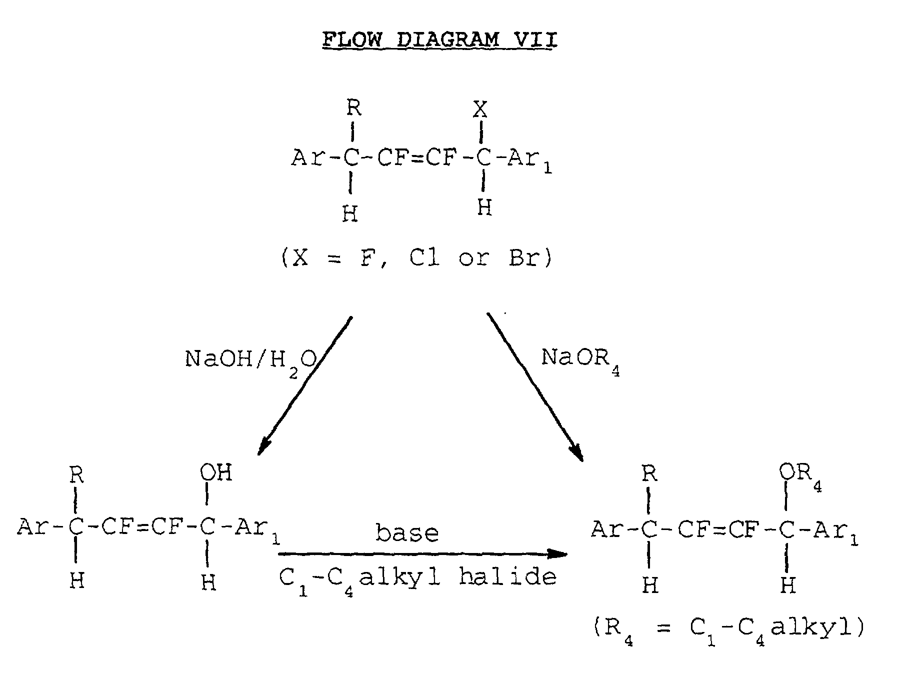

Starting formula II compounds may be prepared, as shown in Flow Diagram VIII, by sequentially reacting bromotrifluoroethylene with zinc, copper(I) bromide and a substituted methyl bromide of formula VII or a trifluoroacetate of formula VIII.

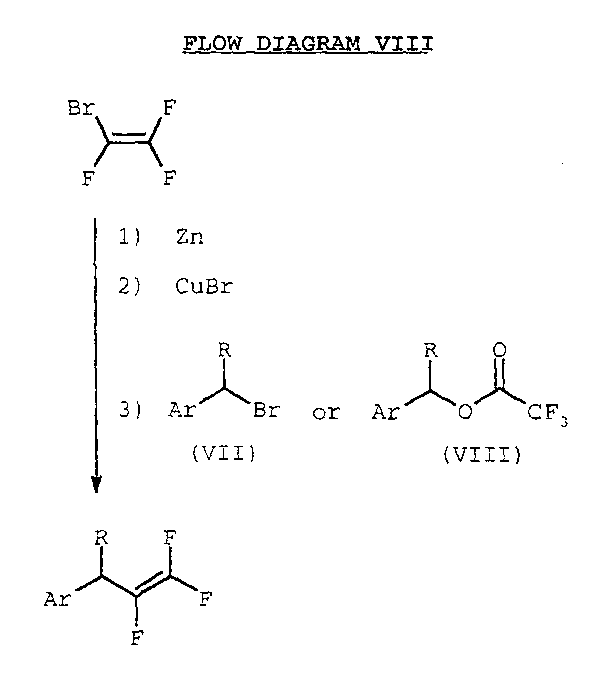

Intermediate compounds of formula VII may be prepared, as illustrated in Flow Diagram IX, by reacting an arylmagnesium bromide compound of formula IX with an aldehyde of formula X and a mineral acid to form an alcohol of formula XI, and reacting the formula XI alcohol with hydrobromic acid.

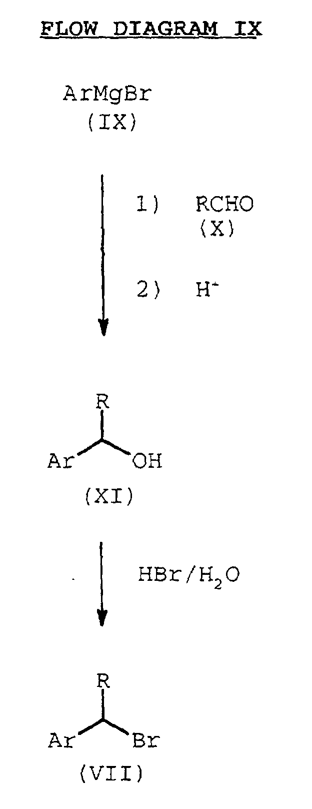

Compounds of formula VIII may be prepared by reacting a formula XI alcohol with trifluoroacetic anhydride. The reaction scheme is shown in Flow Diagram X.

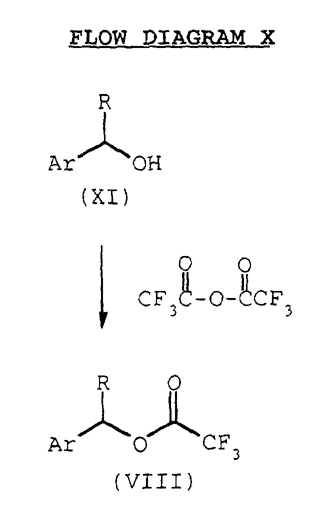

Intermediate alcohols of formula XI may also be prepared, as shown in Flow Diagram XI, by reacting an aryl bromide of formula XII with an alkyllithium such as *n*-butyllithium and an aldehyde of formula X.

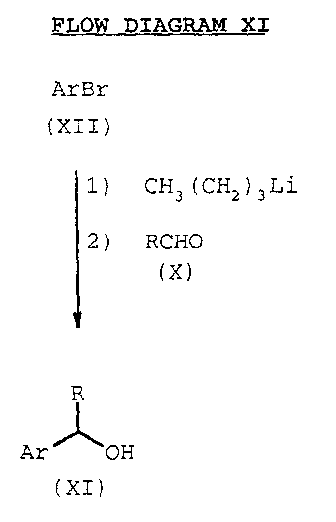

Other methods for the preparation of formula I compounds will become apparent from the examples set forth below.

The present invention also relates to intermediate compounds having the structural formula XIII 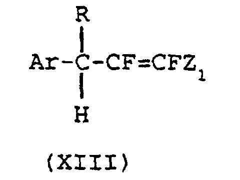 wherein
- Ar: is phenyl optionally substituted with any combination of from one to three halogen, C₁-C₄alkyl, C₁-C₄haloalkyl, C₁-C₄alkoxy or C₁-C₄haloalkoxy groups,
1- or 2-naphthyl optionally substituted with any combination of from one to three halogen, C₁-C₄alkyl, C₁-C₄haloalkyl, C₁-C₄alkoxy or C₁-C₄haloalkoxy groups, or
a 5- or 6-membered heteroaromatic ring optionally substituted with any combination of from one to three halogen, C₁-C₄alkyl, C₁-C₄haloalkyl, C₁-C₄alkoxy or C₁-C₄haloalkoxy groups;
- R: is hydrogen, C₁-C₄alkyl, C₃-C₆cycloalkyl or C₃-C₆halocycloalkyl; and
- Z₁: is hydrogen or F, and
the optical isomers thereof, and the (E)- and (Z)- isomers thereof.

Preferred intermediate compounds of formula XIII are those wherein
- Ar: is phenyl optionally substituted with any combination of from one to three halogen, C₁-C₄alkyl, C₁-C₄haloalkyl, C₁-C₄alkoxy or C₁-C₄haloalkoxy groups;
- R: is C₁-C₄alkyl, C₃-C₆cycloalkyl or
C₃-C₆halocycloalkyl; and
- Z₁: is hydrogen or F.

More preferred formula XIII compounds are those wherein
- Ar: is phenyl optionally substituted with any combination of from one to three halogen, C₁-C₄alkyl, C₁-C₄haloalkyl, C₁-C₄alkoxy or C₁-C₄haloalkoxy groups;
- R: is isopropyl; and
- Z₁: is hydrogen or F.

The invention also provides a process for the preparation of intermediate compounds of formula XIII which comprises:
a) reacting bromotrifluoroethylene with a substituted methyl halide of formula VII' 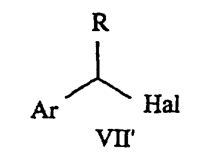 for example with a methyl bromide,
   or a trifluoroacetate of formula VIII 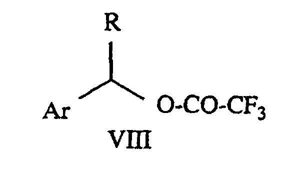 to provide the desired compound of formula XIII wherein Z₁ is fluoro, for example using sodium bis(2-methoxyethoxy) aluminum hydride and a mineral acid such as hydrochloric acid,
or b) by converting a compound of formula XIII wherein Z₁ is fluoro to the desired compound of formula XIII wherein Z₁ is hydrogen, for example by sequentially reacting bromotrifluoroethylene with zinc, copper(I) bromide and then the substituted methyl bromide of formula VII or the trifluoroacetate of formula VIII.

The 1,4-diaryl-2,3-difluoro-2-butene compounds of the present invention are effective for controlling insect and acarid pests. Those compounds are also effective for protecting growing or harvested crops from damage caused by insect and acarid attack and infestation.

Insects controlled by the 1,4-diaryl-2,3-difluoro-2-butene compounds of this invention include Lepidoptera such as tobacco budworms, cabbage loopers, cotton boll worms, beet armyworms, southern armyworms and diamondback moths; Homoptera such as aphids, leaf hoppers, plant hoppers and white flies; Thysanoptera such as thrips; Coleoptera such as boll weevils, Colorado potato beetles, southern corn rootworms, western corn rootworms and mustard beetles; and Orthoptera such as locusts, crickets, grasshoppers and cockroaches. Acarina controlled by the compounds of this invention include mites such as two-spotted spider mites, carmine spider mites, banks grass mites, strawberry mites, citrus rust mites and leprosis mites.

In practice generally about 10 ppm to about 10,000 ppm and preferably about 100 ppm to about 5,000 ppm of a formula I compound, dispersed in water or another liquid carrier, is effective when applied to plants or the soil in which the plants are growing to protect the plants from insect and acarid attack and infestation.

The 1,4-diaryl-2,3-difluoro-2-butene compounds of this invention are also effective for controlling insect and acarid pests when applied to the foliage of plants and/or to the soil or water in which said plants are growing in sufficient amount to provide a rate of about 0.1 kg/ha to 4.0 kg/ha of active ingredient.

While the compounds of this invention are effective for controlling insect and acarid pests when employed alone, they may also be used in combination with other biological chemicals, including other insecticides and acaricides. For example, the formula I compounds of this invention may be used effectively in conjunction or combination with pyrethroids, phosphates, carbamates, cyclodienes, endotoxin of *Bacillus thuringiensis* (Bt), formamidines, phenol tin compounds, chlorinated hydrocarbons, benzoylphenyl ureas, pyrroles and the like.

The compounds of this invention may be formulated as emulsifiable concentrates, flowable concentrates or wettable powders which are diluted with water or other suitable polar solvent, generally *in situ,* and then applied as a dilute spray. Said compounds may also be formulated in dry compacted granules, granular formulations, dusts, dust concentrates, suspension concentrates, microemulsions and the like all of which lend themselves to seed, soil, water and/or foliage applications to provide the requisite plant protection. Such formulations or compositions of the present invention include a compound of the invention (or combinations thereof) admixed with one or more agronomically acceptable inert, solid or liquid carriers. Those compositions contain a pesticidally effective amount of said compound or compounds, which amount may vary depending upon the particular compound, target pest, and method of use. Those skilled in the art can readily determine what is a pesticidally effective amount without undue experimentation.

In order to facilitate a further understanding of the invention, the following examples are presented primarily for the purpose of illustrating more specific details thereof. The scope of the invention should not be deemed limited by the examples, but encompasses the entire subject matter defined in the claims.

### EXAMPLE 1: Preparation of p-Chloro-α-isopropylbenzyl alcohol

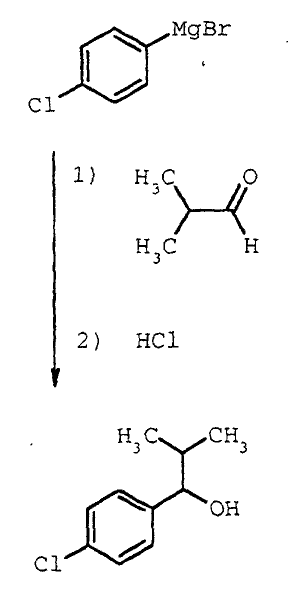

A 1 M solution of p-chlorophenylmagnesium bromide in diethyl ether (100 mL) is added to a solution of isobutyraldehyde (9.08 mL, 0.1 mol) in diethyl ether at -5 °C. °After the addition is complete, the reaction mixture is stirred overnight at room temperature, diluted with an ice-water mixture, and acidified with 10% hydrochloric acid. The phases are separated and the aqueous phase is extracted with diethyl ether. The organic phase and extracts are combined, washed sequentially with saturated sodium hydrogen carbonate solution, water and brine, dried over anhydrous sodium sulfate, and concentrated *in vacuo* to obtain an oil. Column chromatography of the oil using silica gel and a 33% methylene chloride in hexanes solution gives the title product as a colorless oil (14 g) which is identified by NMR spectral analyses.

Using essentially the same procedure, the following compounds are obtained:

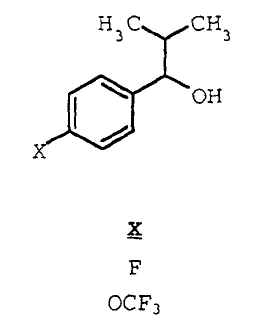

### EXAMPLE 2: Preparation of 1-(1-Bromo-2-methylpropyl)-4-chloro-benzene

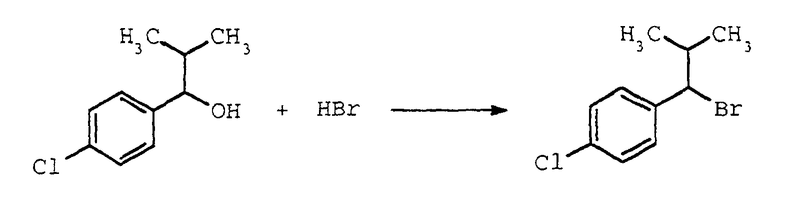

A mixture of *p*-chloro-α-isopropylbenzyl alcohol (33.08 g) in 48% hydrobromic acid (330 mL) is stirred at room temperature for 1 hour and extracted with hexanes. The combined organic extracts are washed sequentially with water, saturated sodium hydrogen carbonate solution and water, dried over anhydrous sodium sulfate, and concentrated *in vacuo* to give the title product as a yellow oil (42.98 g) which is identified by NMR spectral analysis.

Using essentially the same procedure, the following compounds are obtained:

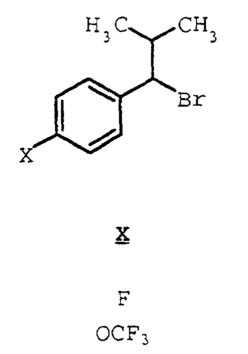

### EXAMPLE 3: Preparation of 3-(p-Chlorophenyl)-1,1,2-trifluoro-4-methyl-1-pentene

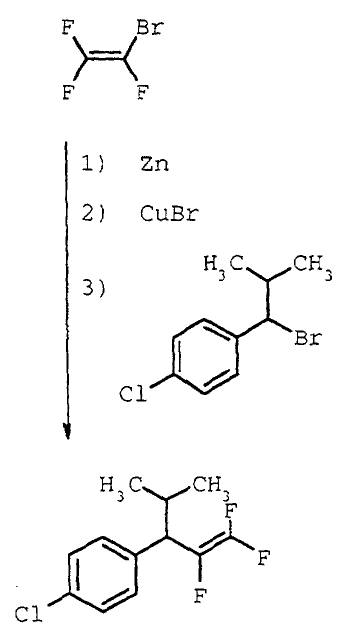

A mixture of zinc dust (15.72 g) and bromotrifluoroethylene (48.34 g, 0.30 mol) in N,N-dimethylformamide is heated to 38°C. After stirring at 38 °C for several minutes, the reaction mixture temperature rises to 65°C over 30 minutes. The reaction mixture is then stirred for 90 minutes and cooled to -5°C. Copper(I) bromide (34.5 g, 0.24 mol) is then added to the cooled mixture. The reaction mixture is stirred at room temperature for 1 hour, treated sequentially with 1-(1-bromo-2-methylpropyl)-4-chlorobenzene (18.56 g, 0.075 mol) and limonene (5 drops), stirred at 49 °C overnight, cooled, and diluted with saturated ammonium chloride solution (400 mL) and concentrated ammonia solution (100 mL). The resultant aqueous mixture is extracted with hexanes. The combined organic extracts are washed sequentially with water, 10% hydrochloric acid, water, saturated sodium hydrogen carbonate solution and water, dried over anhydrous sodium sulfate, and concentrated *in vacuo* to obtain an oil. Column chromatography of the oil using silica gel and hexanes gives the title product as an oil (5.54 g) which is identified by NMR spectral analyses.

Using essentially the same procedure, the following compounds are obtained:

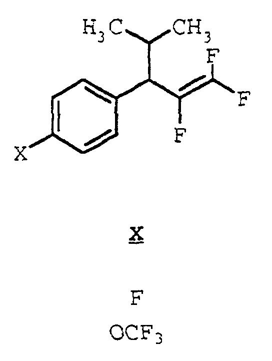

In addition, when 1-(*p*-ethoxyphenyl)-2-methylpropyl trifluoroacetate and 1-(*p*-methoxyphenyl)-2-methylpropyl trifluoroacetate are substituted for 1-(1-bromo-2-methylpropyl)-4-chlorobenzene, 3-(*p*-ethoxyphenyl)-1,1,2-trifluoro-4-methyl-1-pentene and 3-(*p*-methoxyphenyl)-1,1,2-trifluoro-4-methyl-1-pentene are obtained, respectively.

### EXAMPLE 4: Preparation of 3-(p-Chlorophenyl)-1,2-difluoro-4-methyl-1-pentene

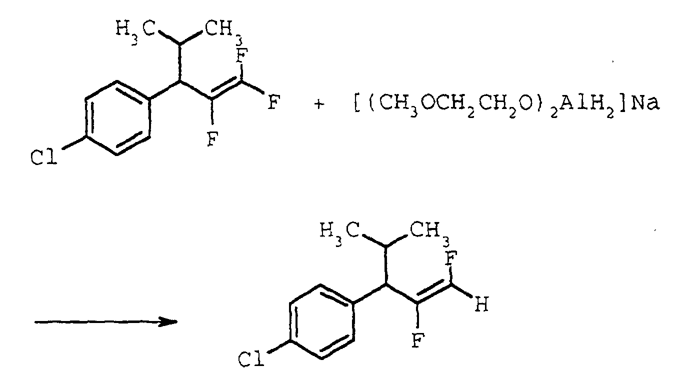

A solution of 3-(*p*-chlorophenyl)-1,1,2-trifluoro-4-methyl-1-pentene (2.48 g, 0.01 mol) in tetrahydrofuran is cooled to -8°C, treated dropwise with a 3.4 M solution of sodium bis(2-methoxyethoxy)aluminum hydride in toluene (3.1 mL), stirred overnight at room temperature, diluted with water, acidified with 10% hydrochloric acid, and extracted with methylene chloride. The combined organic extracts are washed sequentially with water, saturated sodium hydrogen carbonate solution and water, dried over anhydrous sodium sulfate, and concentrated *in vacuo* to obtain the title product as a yellow oil (2.10 g) which is identified by NMR spectral analyses.

Using essentially the same procedure, the following compounds are obtained:

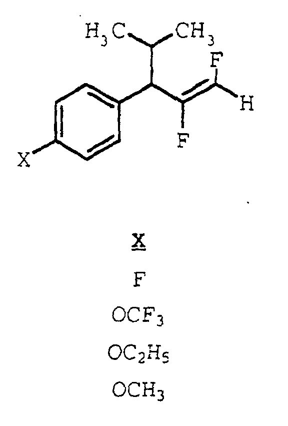

### EXAMPLE 5: Preparation of 4-(p-Chlorophenyl)-2,3-difluoro-5-methyl-1-(m-phenoxyphenyl)-2-hexene, (E)-

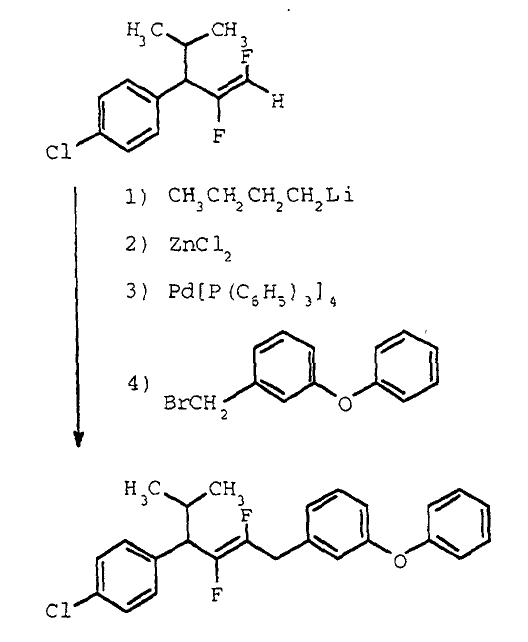

A solution of 3-(*p*-chlorophenyl)-1,2-difluoro-4-methyl-1-pentene (0.69 g, 0.003 mol) in tetrahydrofuran is cooled to -70°C, treated with a 2.5 M solution of *n*-butyllithium in hexane (1.2 mL), stirred at -60°C for 1 hour, treated with a 0.5 M solution of zinc chloride in tetrahydrofuran (6 mL), stirred at -60°C for 1 hour, treated sequentially with a solution of tetrakis(triphenylphosphine)palladium(0) (0.081 g) in tetrahydrofuran and a solution of α-bromo-*m*-tolyl phenyl ether (0.789 g, 0.003 mol) in tetrahydrofuran, stirred at room temperature overnight, diluted with water, acidified with 10% hydrochloric acid, and extracted with methylene chloride. The combined organic extracts are washed with water, dried over anhydrous sodium sulfate, and concentrated *in vacuo* to obtain an oil. Column chromatography of the oil using silica gel and pentane affords an oil which is purified by Kugelrohr distillation to give the title product as a pale, yellow oil (0.46 g) which is identified by NMR spectral analyses.

Using essentially the same procedure, the following compounds are obtained:

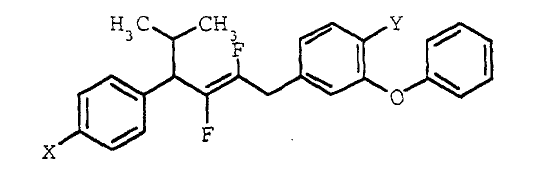

| **X** | **Y** | **state** |
|---|---|---|
| Cl | F | colorless oil |
| OCF₃ | H | pale, yellow oil |
| OCF₃ | F | yellow oil |
| F | H | yellow oil |
| F | F | yellow oil |
| OC₂H₅ | H | colorless oil |
| OC₂H₅ | F | pale, yellow oil |
| OCH₃ | H | brown oil |

### EXAMPLE 6: Preparation of p-Etoxy-α-isopropylbenzyl alcohol

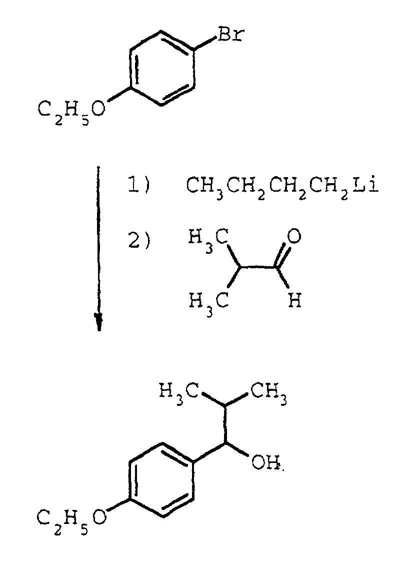

A solution of *p*-bromophenetole (2.01 g, 0.01 mol) in tetrahydrofuran is cooled to -65°C, treated dropwise with a 2.5 M solution of *n*-butyllithium in hexane (4 mL), stirred for 20 minutes at -55 to -65°C, treated dropwise with a solution of isobutyraldehyde (0.91 mL, 0.01 mol) in tetrahydrofuran, stirred overnight at room temperature, diluted with an ice-water mixture, acidified with 10% hydrochloric acid, and extracted with methylene chloride. The combined organic extracts are washed sequentially with water, saturated sodium hydrogen carbonate solution and water, dried over anhydrous sodium sulfate, and concentrated *in vacuo* to give the title product as a tan oil (1.95 g) which is identified by NMR spectral analyses.

Using essentially the same procedure, the following compound is obtained:

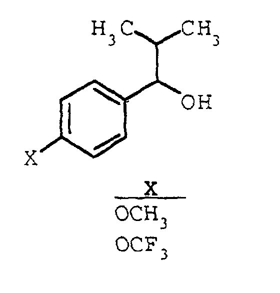

### EXAMPLE 7: Preparation of 1-(p-Ethoxyphenyl)-2-methylpropyl trifluoroacetate

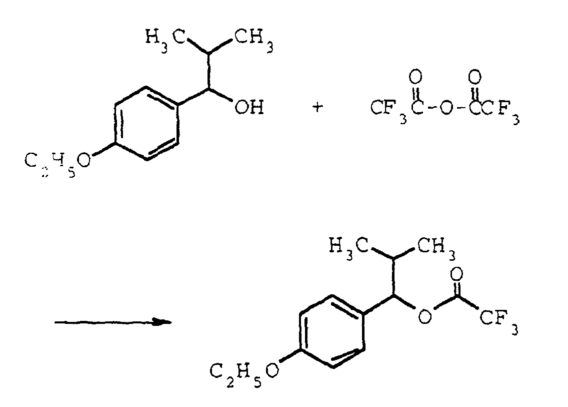

A mixture of trifluoroacetic anhydride (18 mL) in carbon tetrachloride is cooled with an ice-water bath, treated portionwise with a solution of *p*-ethoxy-α-isopropylbenzyl alcohol (9.0 g) in carbon tetrachloride, stirred at room temperature for 1 hour, concentrated *in vacuo,* diluted with carbon tetrachloride, and concentrated *in vacuo* to give the title product as a brown oil (13.23 g) which is identified by NMR spectral analysis.

Using essentially the same procedure, the following compound is obtained:

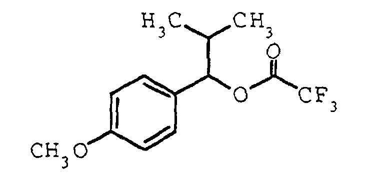

### EXAMPLE 8: Preparation of p-[2,3-Difluoro-1-isopropyl-4-(m-phenoxyphenyl)-2-buten-1-yl]phenol, (E)-

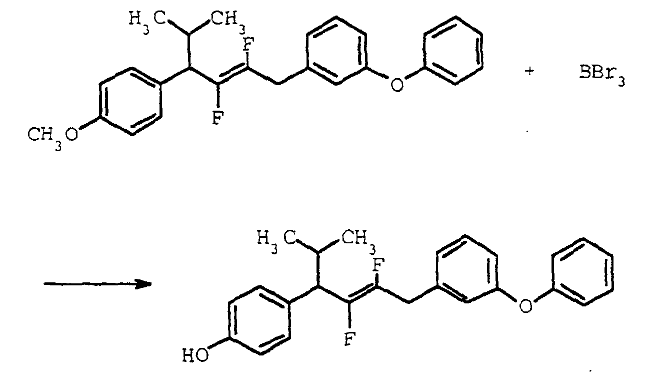

A solution of 2,3-difluoro-4-(*p*-methoxyphenyl)-5-methyl-1-(*m*-phenoxyphenyl)-2-hexene, (E)- (0.408 g, 0.001 mol) in methylene chloride is cooled to -10°C, treated dropwise with a 1 M solution of boron tribromide in methylene chloride (1.0 mL, 0.001 mol), stirred overnight at room temperature, cooled, diluted with methanol, and concentrated *in vacuo* to obtain a residue. The residue is dissolved in methylene chloride. The resultant solution is washed sequentially with saturated sodium hydrogen carbonate solution and water, dried over anhydrous sodium sulfate, and concentrated *in vacuo* to obtain a brown oil. Column chromatography of the oil using silica gel and methylene chloride gives the title product as a pale, yellow oil (0.355 g) which is identified by NMR spectral analyses.

### EXAMPLE 9: Preparation of 4-[p-(Difluoromethoxy) phenyl]-2,3-difluoro-5-methyl-1-(m-phenoxyphenyl)-2-hexene, (E)-

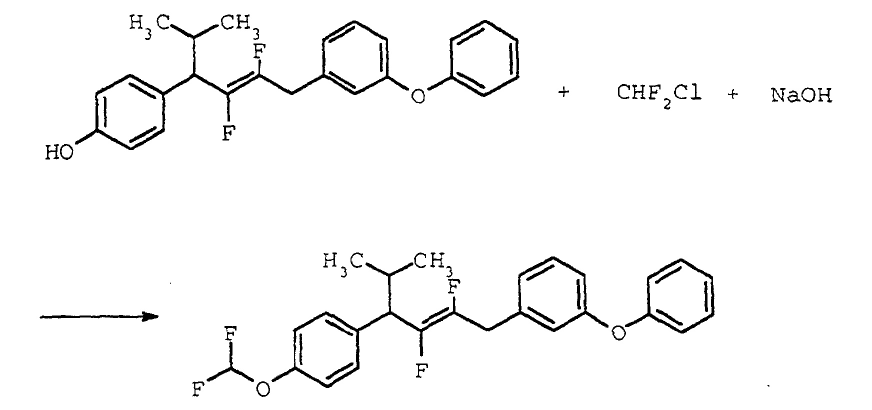

A mixture of p-[2,3-difluoro-1-isopropyl-4-(*m*-phenoxyphenyl)-2-buten-1-yl]phenol, (E)- (0.173 g), dioxane (6 mL) and water (4.5 mL) is treated sequentially with difluorochloromethane (70 drops) and sodium hydroxide (0.217 g), stirred at 70°C for 1 hour, cooled, treated with additional difluorochloromethane (50 drops) and sodium hydroxide (0.245 g), heated at 58-66°C for 3 hours, cooled, treated with additional difluorochloromethane (50 drops) and sodium hydroxide (0.185 g), heated for an additional 2.5 hours, stirred overnight at room temperature, and diluted with water. The resultant aqueous mixture is extracted with methylene chloride. The combined organic extracts are washed with water, dried over anhydrous sodium sulfate, and concentrated *in vacuo* to obtain a light brown oil. Column chromatography of the oil using silica gel and methylene chloride gives the title product as a colorless oil (0.134 g) which is identified by NMR spectral analyses.

### EXAMPLE 10: Insecticidal and acaricidal evaluation of test compounds

Test solutions are prepared by dissolving the test compound in a 35% acetone in water mixture to give a concentration of 10,000 ppm. Subsequent dilutions are made with water as needed.

### Spodoptera eridania, 3rd instar larvae, southern armyworm (SAW)

A Sieva lima bean leaf expanded to 7-8 cm in length is dipped in the test solution with agitation for 3 seconds and allowed to dry in a hood. The leaf is then placed in a 100 x 10 mm petri dish containing a damp filter paper on the bottom and ten 3rd instar caterpillars. At 5 days, observations are made of mortality, reduced feeding, or any interference with normal molting.

### Diabrotica virgifera virgifera Leconte, 3rd instar western corn rootworm (WCR)

One cc of fine talc is placed in a 30 mL wide-mouth screw-top glass jar. One mL of the appropriate acetone test solution is pipetted onto the talc so as to provide 1.25 mg of active ingredient per jar. The jars are set under a gentle air flow until the acetone is evaporated. The dried talc is loosened, 1 cc of millet seed is added to serve as food for the insects and 25 mL of moist soil is added to each jar. The jar is capped and the contents thoroughly mixed mechanically. Following this, ten 3rd instar rootworms are added to each jar and the jars are loosely capped to allow air exchange for the larvae. The treatments are held for 5 days when mortality counts are made. Missing larvae are presumed dead, since they decompose rapidly and cannot be found. The concentrations of active ingredient used in this test correspond approximately to 50 kg/ha.

### Heliothis virenscens, 3rd instar tobacco budworm (TBW)

Cotton cotyledons are dipped in the test solution and allowed to dry in a hood. When dry, each is cut into quarters and ten sections are placed individually in 30 mL plastic medicine cups containing a 5 to 7 mm long piece of damp dental wick. One 3rd instar caterpillar is added to each cup and a cardboard lid placed on the cup. Treatments are maintained for 3 days before mortality counts and estimates of reduction in feeding damage are made.

### Aphis fabae, mixed instar, bean aphid (BA)

Pots containing single nasturtium plants (Tropaeolum sp.) about 5 cm tall are infested with about 100-200 aphids one day before the test. Each pot is sprayed with the test solution for 2 revolutions of a 4 rpm turntable in a hood. The spray is directed to give complete coverage of the plants and aphids. The sprayed pots are set on their sides on white trays and held for 2 days, following which mortality estimates are made.

### Tetranychus urticae (OP-resistant strain), 2-spotted spider mite (TSM)

Sieva lima bean plants with primary leaves expanded to 7-8 cm are selected and cut back to one plant per pot. A small piece is cut from an infested leaf taken from the main colony and placed on each leaf of the test plants. This is done about 2 hours before treatment to allow the mites to move over to the test plant to lay eggs. The size of the cut, infested leaf is varied to obtain about 100 mites per leaf. At the time of test treatment, the piece of leaf used to transfer the mites is removed and discarded. The newly-infested plants are dipped in the test solution for 3 seconds with agitation and set in the hood to dry. After 2 days, one leaf is removed and mortality counts are made.

The tests are rated according to the scale shown below and the data obtained are shown in Table I.

Compounds employed in the above-described evaluations are given a compound number and identified by name. Data in Table I are reported by compound number.

| **Rating Scale** | |
|---|---|
| 0 = no effect | 5 = 56-65% kill |
| 1 = 10-25% kill | 6 = 66-75% kill |
| 2 = 26-35% kill | 7 = 76-85% kill |
| 3 = 36-45% kill | 8 = 86-99% kill |
| 4 = 46-55% kill | 9 = 100% kill |

### COMPOUNDS EVALUATED AS INSECTICIDAL AND ACARICIDAL AGENTS

| Compound Number | |
|---|---|
| 1 | 4-(*p*-Chlorophenyl)-2,3-difluoro-5-methyl-1-(*m*-phenoxyphenyl)-2-hexene, (E)- |
| 2 | 4-(*p*-Chlorophenyl)-2,3-difluoro-1-(4-fluoro-3-phenoxyphenyl)-5-methyl-2-hexene, (E)- |
| 3 | 2,3-Difluoro-5-methyl-1-(*m*-phenoxyphenyl)-4-[*p*-(trifluoromethoxy)phenyl]-2-hexene, (E)- |
| 4 | 4-(*p*-Ethoxyphenyl)-2,3-difluoro-1-(*m*-phenoxyphenyl)-5-methyl-2-hexene, (E)- |
| 5 | 4-(*p*-Ethoxyphenyl)-2,3-difluoro-1-(4-fluoro-3-phenoxyphenyl)-5-methyl-2-hexene, (E)- |
| 6 | 2,3-Difluoro-1-(4-fluoro-3-phenoxyphenyl)-5-methyl-4-[*p*-(trifluoromethoxy)phenyl]-2-hexene, (E) - |
| 7 | 2,3-Difluoro-4-(*p*-fluorophenyl)-5-methyl-1-(*m*-phenoxyphenyl)-2-hexene, (E)- |
| 8 | 2,3-Difluoro-1-(4-fluoro-3-phenoxyphenyl)-4-(*p*-fluorophenyl)-5-methyl-2-hexene, (E)- |

**TABLE I**

| **Insecticidal And Acaricidal Evaluations** | | | | | |
|---|---|---|---|---|---|
| Compound Number | SAW | WCR | TEW | BA | TSM |
| | (100 ppm) | (50 ppm) | (100 ppm) | (100 ppm) | (100 ppm) |
| 1 | 9 | 9 | 9 | 4 | 0 |
| 2 | 9 | 9 | 9 | 7 | 0 |
| 3 | 9 | 9 | 9 | 2 | 5 |
| 4 | 9 | 9 | 9 | 8 | 0 |
| 5 | 9 | 9 | 9 | 7 | 0 |
| 6 | 9 | 9 | 9 | 8 | 9 |
| 7 | 9 | 9 | 9 | 9 | 0 |
| 8 | 9 | 9 | 9 | 8 | 7 |

## Claims

1. A compound of formula I 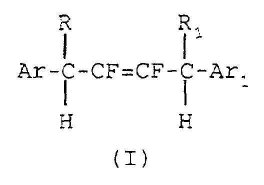 wherein
Ar is phenyl optionally substituted with any combination of from one to three halogen, C₁-C₄alkyl, C₁-C₄haloalkyl, C₁-C₄alkoxy, C₁-C₄haloalkoxy or hydroxy groups,
1- or 2-naphthyl optionally substituted with any combination of from one to three halogen, C₁-C₄alkyl, C₁-C₄haloalkyl, C₁-C₄alkoxy or C₁-C₄haloalkoxy groups, or
a 5- or 6-membered heteroaromatic ring optionally substituted with any combination of from one to three halogen, C₁-C₄alkyl, C₁-C₄haloalkyl, C₁-C₄alkoxy or C₁-C₄haloalkoxy groups;
R is hydrogen, C₁-C₄alkyl, C₁-C₄haloalkyl, C₃-C₆cycloalkyl or C₃-C₆halocycloalkyl;
R₁ is hydrogen, F, Cl, Br, cyano or OR₂;
R₂ is hydrogen or C₁-C₄alkyl; and
Ar₁ is phenoxyphenyl optionally substituted with any combination of from one to six halogen, C₁-C₄alkyl, C₁-C₄haloalkyl, C₁-C₄alkoxy or C₁-C₄haloalkoxy groups,
phenyl optionally substituted with any combination of from one to five halogen, C₁-C₄alkyl, C₁-C₄haloalkyl, C₁-C₄alkoxy or C₁-C₄haloalkoxy groups,
biphenyl optionally substituted with any combination of from one to five halogen, C₁-C₄alkyl, C₁-C₄haloalkyl, C₁-C₄alkoxy or C₁-C₄haloalkoxy groups,
phenoxypyridyl optionally substituted with any combination of from one to five halogen, C₁-C₄alkyl, C₁-C₄haloalkyl, C₁-C₄alkoxy or C₁-C₄haloalkoxy groups,
benzylpyridyl optionally substituted with any combination of from one to five halogen, C₁-C₄alkyl, C₁-C₄haloalkyl, C₁-C₄alkoxy or C₁-C₄haloalkoxy groups,
benzylphenyl optionally substituted with any combination of from one to five halogen, C₁-C₄alkyl, C₁-C₄haloalkyl, C₁-C₄alkoxy or C₁-C₄haloalkoxy groups,
benzoylphenyl optionally substituted with any combination of from one to five halogen, C₁-C₄alkyl, C₁-C₄haloalkyl, C₁-C₄alkoxy or C₁-C₄haloalkoxy groups,
1- or 2-naphthyl optionally substituted with any combination of from one to three halogen, C₁-C₄alkyl, C₁-C₄haloalkyl, C₁-C₄alkoxy or C₁-C₄haloalkoxy groups, or
a 5- or 6-membered heteroaromatic ring optionally substituted with any combination of from one to three halogen, C₁-C₄alkyl, C₁-C₄haloalkyl, C₁-C₄alkoxy or C₁-C₄haloalkoxy groups, and
the optical isomers thereof, and the (E)- and (Z)- isomers thereof.

2. A compound as claimed in claim 1 wherein
Ar is phenyl optionally substituted with any combination of from one to three halogen, C₁-C₄alkyl, C₁-C₄haloalkyl, C₁-C₄alkoxy or C₁-C₄haloalkoxy groups;
R is C₁-C₄alkyl, C₁-C₄haloalkyl, C₃-C₆cycloalkyl or C₃-C₆halocycloalkyl; and
Ar₁ is 3-phenoxyphenyl optionally substituted with any combination of from one to six halogen, C₁-C₄alkyl, C₁-C₄haloalkyl, C₁-C₄alkoxy or C₁-C₄haloalkoxy groups,
3-biphenyl optionally substituted with any combination of from one to five halogen, C₁-C₄alkyl, C₁-C₄haloalkyl, C₁-C₄alkoxy or C₁-C₄haloalkoxy groups, or
3-benzylphenyl optionally substituted with any combination of from one to five halogen, C₁-C₄alkyl, C₁-C₄haloalkyl, C₁-C₄alkoxy or C₁-C₄haloalkoxy groups.

3. A compound as claimed in claim 2 wherein
R is isopropyl, trifluoromethyl or cyclopropyl;
R₁ is hydrogen; and
Ar₁ is 3-phenoxyphenyl optionally substituted with any combination of from one to six halogen, C₁-C₄alkyl, C₁-C₄haloalkyl, C₁-C₄alkoxy or C₁-C₄haloalkoxy groups.

4. A compound as claimed in claim 3 wherein R is isopropyl.

5. A compound as claimed in claim 4 selected from the group consisting of
4-(*p*-chlorophenyl)-2,3-difluoro-5-methyl-1-(*m*-phenoxyphenyl)-2-hexene, (E)-;
4-(*p*-chlorophenyl)-2,3-difluoro-1-(4-fluoro-3-phenoxyphenyl)-5-methyl-2-hexene, (E)-;
2,3-difluoro-5-methyl-1-(*m*-phenoxyphenyl)-4-[*p*-(trifluoromethoxy)phenyl]-2-hexene, (E)-;
4-(*p*-ethoxyphenyl)-2,3-difluoro-1-(*m*-phenoxyphenyl)-5-methyl-2-hexene, (E)-;
4-(*p*-ethoxyphenyl)-2,3-difluoro-1-(4-fluoro-3-phenoxyphenyl)-5-methyl-2-hexene, (E)-;
2,3-difluoro-1-(4-fluoro-3-phenoxyphenyl)-5-methyl-4-[*p*-(trifluoromethoxy)phenyl]-2-hexene, (E)-;
2,3-difluoro-4-(*p*-fluorophenyl)-5-methyl-1-(*m*-phenoxyphenyl)-2-hexene, (E)-; and
2,3-difluoro-1-(4-fluoro-3-phenoxyphenyl)-4-(*p*-fluorophenyl)-5-methyl-2-hexene, (E)-.

6. A method for the control of insect or acarid pests which comprises contacting said pests or their food supply, habitat or breeding grounds with a pesticidally effective amount of a compound as claimed in any one of claims 1 to 5.

7. A method for the protection of growing plants from attack or infestation by insect or acarid pests which comprises applying to the foliage of the plants, or to the soil or water in which they are growing, a pesticidally effective amount of a compound a claimed in any one of claims 1 to 5.

8. A method as claimed in claim 7 wherein the compound is applied to the plants, or to the soil or water in which they are growing, at a rate of about 0.1 kg/ha to 4.0 kg/ha.

9. A composition for the control of insect or acarid pests which comprises an agronomically acceptable carrier and a pesticidally effective amount of a compound as claimed in any one of claims 1 to 5.

10. A compound having the structural formula 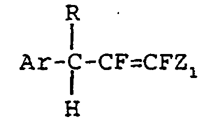 wherein
Ar is phenyl optionally substituted with any combination of from one to three halogen, C₁-C₄ alkyl, C₁-C₄haloalkyl, C₁-C₄alkoxy or C₁-C₄haloalkoxy groups,
1- or 2-naphthyl optionally substituted with any combination of from one to three halogen, C₁-C₄alkyl, C₁-C₄haloalkyl, C₁-C₄alkoxy or C₁-C₄haloalkoxy groups, or
a 5- or 6-membered heteroaromatic ring optionally substituted with any combination of from one to three halogen, C₁-C₄alkyl, C₁-C₄haloalkyl, C₁-C₄alkoxy or C₁-C₄haloalkoxy groups;
R is hydrogen, C₁-C₄alkyl, C₃-C₆cycloalkyl or C₃-C₆halocycloalkyl;
Z₁ is hydrogen or F, and
the optical isomers thereof, and the (E)- and (Z)- isomers thereof.

11. A compound as claimed in claim 10 wherein
Ar is phenyl optionally substituted with any combination of from one to three halogen, C₁-C₄ alkyl, C₁-C₄haloalkyl, C₁-C₄alkoxy or C₁-C₄haloalkoxy groups; and
R is C₁-C₄alkyl, C₃-C₆cycloalkyl or
C₃-C₆halocycloalkyl.

12. A compound as claimed in claim 10 wherein R is isopropyl.

13. A process for the preparation of a compound of formula I as claimed in claim 1 which comprises:
a) converting a compound of formula III to a desired compound of formula I wherein R₁ is H, or OR₂ and R₂ is hydrogen, 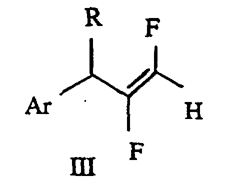
b) converting a compound of formula I wherein R₁is H, or OR₂ and R₂ is hydrogen to a desired compound of formula I wherein R₁ is F, Cl or Br;
c) converting a compound of formula I wherein R₁ is F, Cl or Br to a desired compound of formula I wherein R₁ is CN or OR₂ and R₂ is hydrogen or C₁-C₄alkyl;
or d) converting a compound of formula VI wherein R₃ is SO₂CH₃ or SO₂C₆H₄-4-CH₃ to a desired compound of formula I wherein R₁ is CN

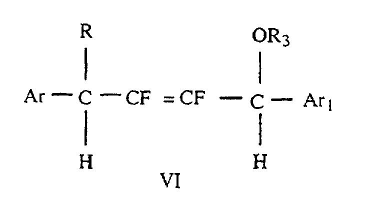

14. A process for the preparation of a compound having the structural formula 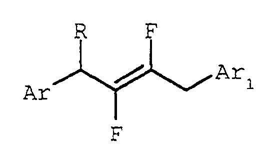 wherein Ar, Ar₁, and R are as defined in claim 1,
which process comprises reacting a 3-aryl-1,1,2-trifluoro-1-propene compound having the structural formula 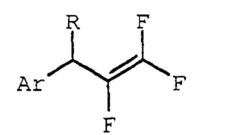 wherein Ar and R are as described above with sodium bis(2-methoxyethoxy)aluminum hydride and a mineral acid to form a 3-aryl-1,2-difluoro-1-propene compound having the structural formula 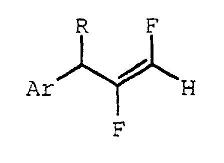 wherein Ar and R are as described above, and sequentially reacting the 3-aryl-1,2-difluoro-1-propene compound with an alkyllithium compound, zinc chloride, tetrakis(triphenylphosphine)palladium(0) and a substituted methyl halide compound having the structural formula
ZCH₂Ar₁
wherein Z is Cl, Br or I and Ar₁ is as described above.

15. The process according to claim 14 wherein
Ar is phenyl optionally substituted with any combination of from one to three halogen, C₁-C₄alkyl, C₁-C₄haloalkyl, C₁-C₄alkoxy or C₁-C₄haloalkoxy groups;
R is C₁-C₄alkyl, C₁-C₄haloalkyl, C₃-C₆cycloalkyl or C₃-C₆halocycloalkyl; and
Ar₁ is 3-phenoxyphenyl optionally substituted with any combination of from one to six halogen, C₁-C₄alkyl, C₁-C₄haloalkyl, C₁-C₄alkoxy or C₁-C₄haloalkoxy groups,
3-biphenyl optionally substituted with any combination of from one to five halogen, C₁-C₄alkyl, C₁-C₄haloalkyl, C₁-C₄alkoxy or C₁-C₄haloalkoxy groups, or
3-benzylphenyl optionally substituted with any combination of from one to five halogen, C₁-C₄alkyl, C₁-C₄haloalkyl, C₁-C₄alkoxy or C₁-C₄haloalkoxy groups.

16. The process according to claim 14 wherein
R is isopropyl; and
Ar₁ is 3-phenoxyphenyl optionally substituted with any combination of from one to six halogen, C₁-C₄alkyl, C₁-C₄haloalkyl, C₁-C₄alkoxy or C₁-C₄haloalkoxy groups.

17. A process for the preparation of a compound of formula XIII as claimed in any one of Claims 10 to 12 which comprises:
a) reacting bromotrifluoroethylene with a substituted methyl halide of formula VII' 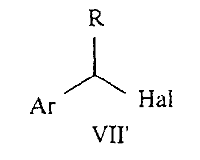 or a trifluoroacetate of formula VIII 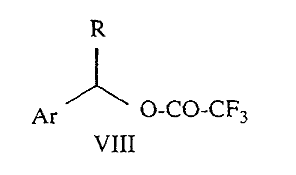 to provide the desired compound of formula XIII wherein Z₁ is fluoro
or b) by converting a compound of formula XIII wherein Z₁ is fluoro to the desired compound of formula XIII wherein Z₁ is hydrogen.

## Patentansprüche

1. Verbindung der Formel I 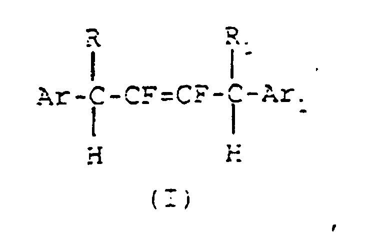
in der Ar Phenyl, das gegebenenfalls mit einer beliebigen Kombination von ein bis drei Halogen-, C₁-C₄Alkyl-, C₁-C₄Halogenalkyl-, C₁-C₄Alkoxy-, C₁-C₄Halogenalkoxy- oder Hydroxygruppen substituiert ist,
1- oder 2-Naphthyl, das gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist, oder
einen 5- oder 6-gliedrigen heteroaromatischen Ring, der gegebenenfalls mit einer beliebigen Kombination von ein bis drei Halogen-, C₁₋C₄Alkyl-, C₁-C₄Halogenalkyl-, C₁-C₄Alkoxy- oder C₁-C₄Halogenalkoxygruppen substituiert ist, bedeutet;
R Wasserstoff, C₁-C₄Alkyl, C₁-C₄Halogenalkyl, C₃-C₆-Cycloalkyl oder C₃₋C₆Halogencycloalkyl bedeutet;
R₁ Wasserstoff, F, Cl, Br, Cyan oder OR₂ bedeutet;
R₂ Wasserstoff oder C₁₋C₄Alkyl bedeutet; und
Ar₁ Phenoxyphenyl, das gegebenenfalls mit einer beliebigen Kombination von ein bis sechs Halogen-, C₁-C₄Alkyl-, C₁-C₄Halogenalkyl-, C₁-C₄Alkoxy- oder C₁-C₄Halogenalkoxygruppen substituiert ist,
Phenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist,
Biphenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist,
Phenoxypyridyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist,
Benzylpyridyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist,
Benzylphenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist,
Benzoylphenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist,
1- oder 2-Naphthyl, das gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist, oder
einen 5- oder 6-gliedrigen heteroaromatischen Ring, der gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist, bedeutet, und
deren optische Isomere und
deren (E)- und (Z)-Isomere.

2. Verbindung nach Anspruch 1, in der
Ar Phenyl, das gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist, bedeutet,
R C₁-C₄Alkyl, C₁-C₄Halogenalkyl, C₃-C₆Cycloalkyl oder C₃₋C₆Halogencycloalkyl bedeutet; und
Ar₁ 3-Phenoxyphenyl, das gegebenenfalls mit einer beliebigen Kombination von ein bis sechs Halogen-, C₁-C₄Alkyl-, C₁-C₄Halogenalkyl-, C₁-C₄Alkoxy- oder C₁-C₄Halogenalkoxygruppen substituiert ist,
3-Biphenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist, oder
3-Benzylphenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist, bedeutet.

3. Verbindung nach Anspruch 2, in der
R Isopropyl, Trifluormethyl oder Cyclopropyl bedeutet;
R₁ Wasserstoff bedeutet; und
Ar₁ 3-Phenoxyphenyl, das gegebenenfalls mit einer beliebigen Kombination von ein bis sechs Halogen-, C₁-C₄Alkyl-, C₁-C₄Halogenalkyl-, C₁-C₄Alkoxy- oder C₁-C₄Halogenalkoxygruppen substituiert ist, bedeutet.

4. Verbindung nach Anspruch 3, in der R Isopropyl bedeutet.

5. Verbindung nach Anspruch 4, aus der Gruppe
(E)-4-(*p*-Chlorphenyl)-2,3-difluor-5-methyl-1-(*m*-phenoxyphenyl)-2-hexene;
(E)-4-(*p*-Chlorphenyl)-2,3-difluor-1-(4-fluor-3-phenoxyphenyl)-5-methyl-2-hexen;
(E)-2,3-Difluor-5-methyl-1-(*m*-phenoxyphenyl)-4-[*p*-(trifluormethoxy)phenyl]-2-hexen;
(E)-4-(*p*-Ethoxyphenyl)-2,3-difluor-1-(*m*-phenoxyphenyl)-5-methyl-2-hexen;
(E)-4- (*p*-Ethoxyphenyl)-2,3-difluor-1-(4-fluor-3-phenoxyphenyl)-5-methyl-2-hexen;
(E)-2,3-Difluor-1-(4-fluor-3-phenoxyphenyl)-5-methyl-4-[*p*-(trifluormethoxy)phenyl]-2-hexen;
(E)-2,3-Difluor-4-(*p*-fluorphenyl)-5-methyl-1-(m-phenoxyphenyl)-2-hexen; und
(E)-2,3-Difluor-1-(4-fluor-3-phenoxyphenyl)-4-(*p*-fluorphenyl)-5-methyl-2-hexen.

6. Verfahren zur Bekämpfung von Schadinsekten oder Schadakariden, **dadurch gekennzeichnet, daß** man diese Schädlinge über ihre Nahrungszufuhr, Umgebung oder Brutstätten mit einer pestizid wirksamen Menge einer Verbindung nach einem der Ansprüche 1 bis 5 in Kontakt bringt.

7. Verfahren zum Schutz von wachsenden Pflanzen gegen Angriff oder Befall durch Schadinsekten oder Schadakariden, **dadurch gekennzeichnet, daß** man eine pestizid wirksame Menge einer Verbindung nach einem der Ansprüche 1 bis 5 auf das Blattwerk der Pflanzen oder auf den Boden oder das Wasser, in dem sie wachsen, ausbringt.

8. Verfahren nach Anspruch 7, wobei man die Verbindung auf die Pflanzen oder auf den Boden oder das Wasser, in dem sie wachsen, in einer Menge von ungefähr 0,1 kg/ha bis 4,0 kg/ha ausbringt.

9. Zusammensetzung zur Bekämpfung von Schadinsekten oder Schadakariden, die einen landwirtschaftlich unbedenklichen Träger und eine pestizid wirksame Menge einer Verbindung nach einem der Ansprüche 1 bis 5 enthält.

10. Verbindung mit der Strukturformel 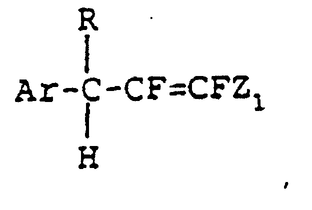
in der Ar Phenyl, das gegebenenfalls mit einer beliebigen Kombination von ein bis drei Halogen-, C₁-C₄Alkyl-, C₁-C₄Halogenalkyl-, C₁-C₄Alkoxy-, C₁-C₄Halogenalkoxygruppen substituiert ist,
1- oder 2-Naphthyl, das gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist, oder
einen 5- oder 6-gliedrigen heteroaromatischen Ring, der gegebenenfalls mit einer beliebigen Kombination von ein bis drei Halogen-, C₁-C₄Alkyl-, C₁-C₄Halogenalkyl-, C₁-C₄Alkoxyoder C₁-C₄Halogenalkoxygruppen substituiert ist, bedeutet;
R Wasserstoff, C₁-C₄Alkyl, C₃-C₆Cycloalkyl oder C₃-C₆Halogencycloalkyl bedeutet;
Z₁ Wasserstoff oder F bedeutet, und
deren optische Isomere und deren (E)- und (Z)-Isomere.

11. Verbindung nach Anspruch 10, in der
Ar Phenyl, das gegebenenfalls mit einer beliebigen Kombination von ein bis drei Halogen-, C₁-C₄Alkyl-, C₁-C₄Halogenalkyl-, C₁-C₄Alkoxy-, C₁-C₄Halogenalkoxygruppen substituiert ist, bedeutet,
R C₁₋C₄Alkyl, C₃-C₆Cycloalkyl oder C₃₋C₆Halogencycloalkyl bedeutet.

12. Verbindung nach Anspruch 10, in der
R Isopropyl bedeutet.

13. Verfahren zur Herstellung einer Verbindung der Formel I nach Anspruch 1, **dadurch gekennzeichnet, daß** man
a) eine Verbindung der Formel III in eine gewünschte Verbindung der Formel I, in der R₁ H bedeutet oder OR₂ und R₂ Wasserstoff bedeuten, umwandelt, 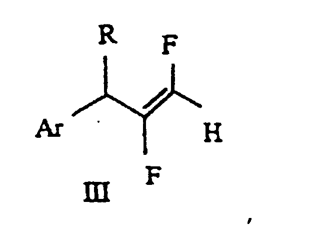
b) eine Verbindung der Formel I, in der R₁ H bedeutet oder OR₂ und R₂ Wasserstoff bedeuten, in eine gewünschte Verbindung der Formel I, in der R₁ F, Cl oder Br bedeutet, umwandelt;
c) eine Verbindung der Formel I, in der R₁ F, Cl oder Br bedeutet, in eine gewünschte Verbindung der Formel I, in der R₁ CN oder OR₂ und R₂ Wasserstoff oder C₁-C₄Alkyl bedeutet;
oder d) eine Verbindung der Formel VI, in der R₃ SO₂CH₃ oder SO₂C₆H₄-4-CH₃ bedeutet, in eine gewünschte Verbindung der Formel I, in der R₁ CN bedeutet, umwandelt.

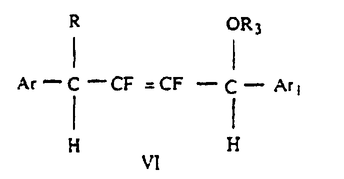

14. Verfahren zur Herstellung einer Verbindung mit der Strukturformel 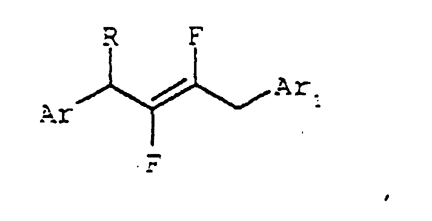 in der Ar, Ar₁ und R wie in Anspruch 1 definiert sind, **dadurch gekennzeichnet, daß** man eine 3-Aryl-1,1,2-trifluor-1-propenverbindung mit der Strukturformel 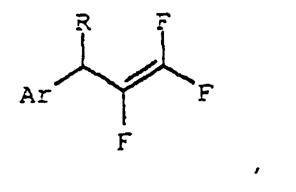 in der Ar und R wie oben beschrieben sind, mit Natriumbis(2-methoxyethoxy)aluminiumhydrid und einer Mineralsäure umsetzt, wodurch man eine 3-Aryl-1,2-difluor-1-propenverbindung mit der Strukturformel 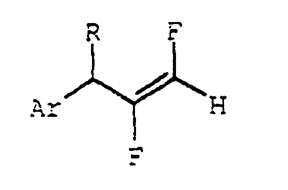 erhält, in der Ar und R wie oben beschrieben sind, und die 3-Aryl-1,2-difluor-1-propenverbindung der nacheinander mit einer Alkyllithiumverbindung, mit Zinkchlorid, mit Tetrakis(triphenylphosphin)-palladium(0) und mit einer substituierten Methylhalogenidverbindung der Strukturformel
ZCH₂Ar₁,
in der Z Cl, Br oder I bedeutet und Ar₁ wie oben beschrieben ist, umsetzt.

15. Verfahren nach Anspruch 14, wobei
Ar Phenyl, das gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist, bedeutet;
R C₁-C₄Alkyl, C₁-C₄Halogenalkyl, C₃-C₆Cycloalkyl oder C₃₋C₆Halogencycloalkyl bedeutet; und
Ar₁ 3-Phenoxyphenyl, das gegebenenfalls mit einer beliebigen Kombination von ein bis sechs Halogen-, C₁-C₄Alkyl-, C₁-C₄Halogenalkyl-, C₁-C₄Alkoxy- oder C₁-C₄Halogenalkoxygruppen substituiert ist,
3-Biphenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist, oder
3-Benzylphenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist, bedeutet.

16. Verfahren nach Anspruch 14, wobei R Isopropyl bedeutet; und
Ar₁ 3-Phenoxyphenyl, das gegebenenfalls mit einer beliebigen Kombination von ein bis sechs Halogen-, C₁-C₄Alkyl-, C₁-C₄Halogenalkyl-, C₁-C₄Alkoxy- oder C₁-C₄Halogenalkoxygruppen substituiert ist, bedeutet.

17. Verfahren zur Herstellung einer Verbindung der Formel XIII nach einem der Ansprüche 10 bis 12, **dadurch gekennzeichnet, daß** man
a) Bromtrifluorethylen mit einem substituierten Methylhalogenid der Formel VII 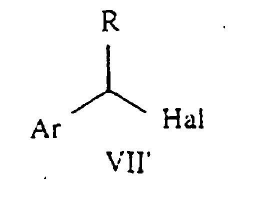 oder einem Trifluoracetat der Formel VIII 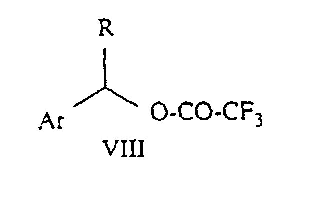 umsetzt, wodurch man die gewünschte Verbindung der Formel XIII, in der Z₁ Fluor bedeutet, erhält
oder b) eine Verbindung der Formel XIII, in der Z₁ Fluor bedeutet, in die gewünschte Verbindung der Formel XIII, in der Z₁ Wasserstoff bedeutet, umwandelt.

## Revendications

1. Composé de formule I 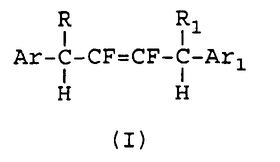 dans laquelle
Ar est un groupe phényle facultativement substitué par toute association de un à trois groupes halogéno, alkyle en C₁-C₄, halogénoalkyle en C₁-C₄, alcoxy en C₁-C₄, halogénoalcoxy en C₁-C₄ ou hydroxyle,
1- ou 2-naphtyle facultativement substitué par toute association de un à trois groupes halogéno, alkyle en C₁-C₄, halogénoalkyle en C₁-C₄, alcoxy en C₁-C₄ ou halogénoalcoxy en C₁-C₄, ou
un cycle hétéroaromatique penta- ou hexagonal facultativement substitué par toute association de un à trois groupes halogéno, alkyle en C₁-C₄, halogénoalkyle en C₁-C₄, alcoxy en C₁-C₄ ou halogénoalcoxy en C₁-C₄ ;
R est l'hydrogène, un groupe alkyle en C₁-C₄, halogénoalkyle en C₁-C₄, cycloalkyle en C₃-C₆ ou halogénocycloalkyle en C₃-C₆ ;
R₁ est l'hydrogène, F, Cl, Br, cyano ou OR₂ ;
R₂ est l'hydrogène ou un groupe alkyle en C₁-C₄ ;
Ar₁ est un groupe phénoxyphényle facultativement substitué par toute association de un à six groupes halogéno, alkyle en C₁-C₄, halogénoalkyle en C₁-C₄, alcoxy en C₁-C₄ ou halogénoalcoxy en C₁-C₄,
phényle facultativement substitué par toute association de un à cinq groupes halogéno, alkyle en C₁-C₄, halogénoalkyle en C₁-C₄, alcoxy en C₁-C₄ ou halogénoalcoxy en C₁-C₄,
biphénylyle facultativement substitué par toute association de un à cinq groupes halogéno, alkyle en C₁-C₄, halogénoalkyle en C₁-C₄, alcoxy en C₁-C₄ ou halogénoalcoxy en C₁-C₄,
phénoxypyridyle facultativement substitué par toute association de un à cinq groupes halogéno, alkyle en C₁-C₄, halogénoalkyle en C₁-C₄, alcoxy en C₁-C₄ ou halogénoalcoxy en C₁-C₄,
benzylpyridyle facultativement substitué par toute association de un à cinq groupes halogéno, alkyle en C₁-C₄, halogénoalkyle en C₁-C₄, alcoxy en C₁-C₄ ou halogénoalcoxy en C₁-C₄,
benzylphényle facultativement substitué par toute association de un à cinq groupes halogéno, alkyle en C₁-C₄, halogénoalkyle en C₁-C₄, alcoxy en C₁-C₄ ou halogénoalcoxy en C₁-C₄,
benzoylphényle facultativement substitué par toute association de un à cinq groupes halogéno, alkyle en C₁-C₄, halogénoalkyle en C₁-C₄, alcoxy en C₁-C₄ ou halogénoalcoxy en C₁-C₄,
1- ou 2-naphtyle facultativement substitué par toute association de un à trois groupes halogéno, alkyle en C₁-C₄, halogénoalkyle en C₁-C₄, alcoxy en C₁-C₄ ou halogénoalcoxy en C₁-C₄, ou
un cycle hétéroaromatique penta- ou hexagonal facultativement substitué par toute association de un à trois groupes halogéno, alkyle en C₁-C₄, halogénoalkyle en C₁-C₄, alcoxy en C₁-C₄ ou halogénoalcoxy en C₁-C₄,
et ses isomères optiques, et ses isomères (E) et (Z).

2. Composé selon la revendication 1, dans lequel
Ar est un groupe phényle facultativement substitué par toute association de un à trois groupes halogéno, alkyle en C₁-C₄, halogénoalkyle en C₁-C₄, alcoxy en C₁-C₄ ou halogénoalcoxy en C₁-C₄,
R est un groupe alkyle en C₁-C₄, halogénoalkyle en C₁-C₄, cycloalkyle en C₃-C₆ ou halogénocycloalkyle en C₃-C₆ ; et
Ar₁ est un groupe 3-phénoxyphényle facultativement substitué par toute association de un à six groupes halogéno, alkyle en C₁-C₄, halogénoalkyle en C₁-C₄, alcoxy en C₁-C₄ ou halogénoalcoxy en C₁-C₄,
3-biphénylyle facultativement substitué par toute association de un à cinq groupes halogéno, alkyle en C₁-C₄, halogénoalkyle en C₁-C₄, alcoxy en C₁-C₄ ou halogénoalcoxy en C₁-C₄, ou
3-benzylphényle facultativement substitué par toute association de un à cinq groupes halogéno, alkyle en C₁-C₄, halogénoalkyle en C₁-C₄, alcoxy en C₁-C₄ ou halogénoalcoxy en C₁-C₄.

3. Composé selon la revendication 2, dans lequel
R est un groupe isopropyle, trifluorométhyle ou cyclopropyle,
R₁ est l'hydrogène ; et
Ar₁ est un groupe 3-phénoxyphényle facultativement substitué par toute association de un à six groupes halogéno, alkyle en C₁-C₄, halogénoalkyle en C₁-C₄, alcoxy en C₁-C₄ ou halogénoalcoxy en C₁-C₄.

4. Composé selon la revendication 3, dans lequel R est un groupe isopropyle.

5. Composé selon la revendication 4, choisi dans la classe formée par les suivants :
4-(*p*-chlorophényl)-2,3-difluoro-5-méthyl-1-(*m*-phénoxyphényl)-2-hexène, (E)- ;
4-(*p*-chlorophényl)-2,3-difluoro-1-(4-fluoro-3-phénoxyphényl)-5-méthyl-2-hexène, (E)- :
2,3-difluoro-5-méthyl-1-(*m*-phénoxyphényl)-4-[*p*-(trifluorométhoxy)phényl]-2-hexène, (E)- ;
4-(*p*-éthoxyphényl)-2,3-difluoro-1-(*m*-phénoxyphényl)-5-méthyl-2-hexène, (E)- ;
4-(*p*-éthoxyphényl)-2,3-difluoro-1-(4-fluoro-3-phénoxyphényl)-5-méthyl-2-hexène, (E)- ;
2,3-difluoro-1-(4-fluoro-3-phénoxyphényl)-5-méthyl-4-[*p*-(trifluorométhoxy)phényl]-2-hexène, (E)- ;
2,3-difluoro-4-(*p*-fluorophényl)-5-méthyl-1-(*m*-phénoxyphényl)-2-hexène, (E)- ; et
2,3-difluoro-1-(4-fluoro-3-phénoxyphényl)-4-(*p*-fluorophényl)-5-méthyl-2-hexène, (E)-.

6. Procédé de lutte contre des insectes et acariens nuisibles, qui consiste à mettre en contact lesdits nuisibles ou leur source de nourriture, leur habitat ou leur site de reproduction avec une quantité à effet pesticide d'un composé tel que revendiqué dans l'une quelconque des revendications 1 à 5.

7. Procédé pour la protection de plantes en croissance contre une attaque ou une infestation par des insectes ou acariens nuisibles, qui consiste à appliquer au feuillage des plantes, ou à la terre ou l'eau dans laquelle elles poussent, une quantité à effet pesticide d'un composé tel que revendiqué dans l'une quelconque des revendications 1 à 5.

8. Procédé selon la revendication 7, dans lequel le composé est appliqué aux plantes ou à la terre ou l'eau dans laquelle elles poussent, à une dose d'environ 0,1 kg/ha à 4,0 kg/ha.

9. Composition pour la lutte contre des insectes ou acariens nuisibles, qui comprend un support agronomiquement acceptable et une quantité à effet pesticide d'un composé tel que revendiqué dans l'une quelconque des revendications 1 à 5.

10. Composé ayant la formule structurale 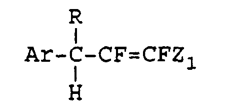 dans laquelle
Ar est un groupe phényle facultativement substitué par toute association de un à trois groupes halogéno, alkyle en C₁-C₄, halogénoalkyle en C₁-C₄, alcoxy en C₁-C₄, halogénoalcoxy en C₁-C₄,
1- ou 2-naphtyle facultativement substitué par toute association de un à trois groupes halogéno, alkyle en C₁-C₄, halogénoalkyle en C₁-C₄, alcoxy en C₁-C₄ ou halogénoalcoxy en C₁-C₄, ou
un cycle hétéroaromatique penta- ou hexagonal facultativement substitué par toute association de un à trois groupes halogéno, alkyle en C₁-C₄, halogénoalkyle en C₁-C₄, alcoxy en C₁-C₄ ou halogénoalcoxy en C₁-C₄ ;
R est l'hydrogène, un groupe alkyle en C₁-C₄, cycloalkyle en C₃-C₆ ou halogénocycloalkyle en C₃-C₆ ;
Z₁ est l'hydrogène ou F,
et ses isomères optiques, et ses isomères (E) et (Z).

11. Composé selon la revendication 10, dans lequel
Ar est un groupe phényle facultativement substitué par toute association de un à trois groupes halogéno, alkyle en C₁-C₄, halogénoalkyle en C₁-C₄, alcoxy en C₁-C₄, halogénoalcoxy en C₁-C₄, et
R est un groupe alkyle en C₁-C₄, cycloalkyle en C₃-C₆ ou halogénocycloalkyle en C₃-C₆.

12. Composé selon la revendication 10, dans lequel R est un groupe isopropyle.

13. Procédé pour la préparation d'un composé de formule I tel que revendiqué dans la revendication 1, qui comprend :
a) la conversion d'un composé de formule III en un composé de formule I désiré dans lequel R₁ est H ou OR₂ et R₂ est l'hydrogène, 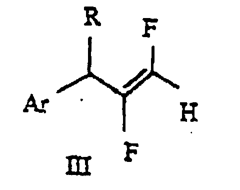
b) la conversion d'un composé de formule I dans lequel R₁ est H ou OR₂ et R₂ est l'hydrogène en un composé de formule I désiré dans lequel R₁ est F, Cl ou Br ;
c) la conversion d'un composé de formule I dans lequel R₁ est F, Cl ou Br en un composé de formule I désiré dans lequel R₁ est CN ou OR₂ et R₂ est l'hydrogène ou un groupe alkyle en C₁-C₄ ; ou
d) la conversion d'un composé de formule VI où R₃ est SO₂CH₃ ou SO₂C₆H₄-4-CH₃ en un composé de formule I désiré dans lequel R₁ est CN

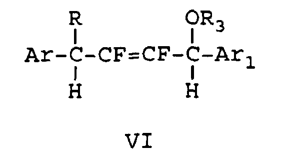

14. Procédé pour la préparation d'un composé ayant la formule structurale 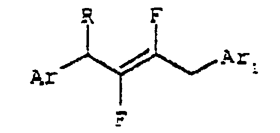 où Ar, Ar₁ et R sont tels que définis dans la revendication 1, lequel procédé comprend la réaction d'un 3-aryl-1,1,2-trifluoro-1-propène ayant la formule structurale 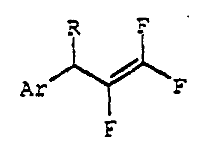 où Ar et R sont tels que définis ci-dessus, avec l'hydrure de sodium et de bis(2-méthoxyéthoxy)aluminium et un acide minéral pour former un 3-aryl-1,2-difluoro-1-propène ayant la formule structurale 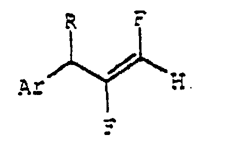 où Ar et R sont tels que définis ci-dessus, puis la réaction du 3-aryl-1,2-difluoro-l-propène avec un alkyl-lithium, du chlorure de zinc, du tétrakis(triphénylphosphine)palladium(0) et un halogénure de méthyle substitué ayant la formule structurale
ZCH₂Ar₁
où Z est Cl, Br ou I et Ar₁ est tel que défini ci-dessus.

15. Procédé selon la revendication 14, dans lequel
Ar est un groupe phényle facultativement substitué par toute association de un à trois groupes halogéno, alkyle en C₁-C₄, halogénoalkyle en C₁-C₄, alcoxy en C₁-C₄, halogénoalcoxy en C₁-C₄ ;
R est un groupe alkyle en C₁-C₄, halogénoalkyle en C₁-C₄, cycloalkyle en C₃-C₆ ou halogénocycloalkyle en C₃-C₆ ; et
Ar₁ est un groupe 3-phénoxyphényle facultativement substitué par toute association de un à six groupes halogéno, alkyle en C₁-C₄, halogénoalkyle en C₁-C₄, alcoxy en C₁-C₄ ou halogénoalcoxy en C₁-C₄,
3-biphénylyle facultativement substitué par toute association de un à cinq groupes halogéno, alkyle en C₁-C₄, halogénoalkyle en C₁-C₄, alcoxy en C₁-C₄ ou halogénoalcoxy en C₁-C₄, ou
3-benzylphényle facultativement substitué par toute association de un à cinq groupes halogéno, alkyle en C₁-C₄, halogénoalkyle en C₁-C₄, alcoxy en C₁-C₄ ou halogénoalcoxy en C₁-C₄.

16. Procédé selon la revendication 14, dans lequel
R est un groupe isopropyle ; et
Ar₁ est un groupe 3-phénoxyphényle facultativement substitué par toute association de un à six groupes halogéno, alkyle en C₁-C₄, halogénoalkyle en C₁-C₄, alcoxy en C₁-C₄ ou halogénoalcoxy en C₁-C₄.

17. Procédé pour la préparation d'un composé de formule XIII tel que revendiqué dans l'une quelconque des revendications 10 à 12, qui comprend :
a) la réaction du bromotrifluoroéthylène avec un halogénure de méthyle de formule VII 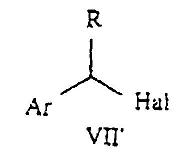 ou un trifluoroacétate de formule VIII 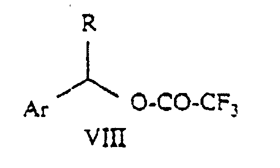 pour produire le composé de formule XIII désiré dans lequel Z₁ est un groupe fluoro, ou
b) la conversion d'un composé de formule XIII dans lequel Z₁ est un groupe fluoro en le composé de formule XIII désiré dans lequel Z₁ est l'hydrogène.
